# EUROPÄISCHE PATENTSCHRIFT

(11) **EP 1 110 458 B1**
(45) Veröffentlichungstag und Bekanntmachung des Hinweises auf die Patenterteilung: **07.12.2005**
(21) Anmeldenummer: 99125398.0
(22) Anmeldetag: 20.12.1999
(51) Int. Cl.: A21D 8/04, C12R 1/225

(54) **Verfahren zur Herstellung eines Sauerteiges unter Verwendung von hetero- und homofermentativen Laktobazillen**
Process for preparing a sourdough with homo- and heterofermentative lactobacillus
Procédé pour la préparation d'un levain de panification à partir de lactobacilles homo- et hétérofermentaires

(43) Veröffentlichungstag der Anmeldung: 27.06.2001
(73) Patentinhaber: Ernst Böcker GmbH & Co. KG, 32427 Minden (DE)
(72) Erfinder: Böcker, Ernst-Joachim, 32427 Minden (DE)
(74) Vertreter: Thiel, Christian

(56) Entgegenhaltungen:
- EP-A- 0 298 605
- EP-A- 0 636 692
- EP-A- 0 953 288
- DE-A- 19 530 890
- FR-A- 2 716 077
- US-A- 5 700 684

## Beschreibung

Die Erfindung betrifft ein Verfahren zur Herstellung von natürlichen Sauerteigen und Sauerteig-Produkten mit hohem Aromapotential und hohem Säuregrad unter Verwendung einer Mischkultur (Mischflora) aus homo- und heterofermentativen Milchsäurebakterien. Die Erfindung betrifft ferner Stämme von Laktobazillen, die für die Durchführung dieses Verfahrens besonders geeignet sind.

Früher diente die Verwendung von Sauerteig in erster Linie der Lockerung des Brotteiges und bei Broten mit überwiegendem Roggenanteil auch der Säuerung des Roggenmehlteiges. Nach der Einführung der Bäckerhefe als Triebmittel zu Beginn dieses Jahrhunderts, diente der Sauerteig bei der Herstellung von Backwaren mit einem Anteil von mehr als 20% Roggenmehl hauptsächlich der Säuerung, da erst durch die Bildung hinreichender Säuremengen die Voraussetzungen für eine elastische und zarte Krume des Roggen-, Roggenmisch-, Weizenmisch- und insbesondere des Schrot- und Vollkorn-Brotes gegeben sind. Eine Sauerteigfermentation ist jedoch nicht auf die bloße Säuerung des Teiges beschränkt. Die Bildung von Aromavorläuferstoffen, die dem Eiweiß-, Fett- und Kohlehydratstoffwechsel der an der Sauerteigfermentation beteiligten Milchsäurebakterien und Hefen entstammen, und die im Backprozess in Aromastoffe umgewandelt werden, tragen wesentlich zum sauerteigtypischen Geschmack und Aroma eines Sauerteigbrotes bei. Ein weiterer positiver Effekt von Sauerteig ist die Unterdrückung des Wachstums von Schimmelpilzen und fadenziehenden Bakterien, wodurch die Haltbarkeit und Lagerstabilität von Sauerteiggebäcken im Vergleich zu ungesäuerten bzw. nicht ausreichend gesäuerten Gebäcken deutlich verbessert ist. Da die Herstellung hochwertiger Sauerteige Zeit- und arbeitsaufwendig ist sowie großer Erfahrung bedarf, werden in Bäckereien häufig kommerziell erworbene getrocknete Sauerteige als Sauerteig-Konzentrate zur Aromatisierung und Säuerung bei der Brotherstellung eingesetzt, um Textur, Aroma, Geschmack und Farbe der Brotkrume und -kruste zu verbessern.

Um eine gleichbleibende Qualität der Backwaren zu gewährleisten, ist es nötig, Sauerteig-Produkte einzusetzen, die über längere Zeit haltbar sind, ohne qualitative Einbußen zu erleiden. Darüber hinaus werden häufig hochkonzentrierte Sauerteig-Produkte eingesetzt, die eine niedrige Dosierung erlauben und damit preislich attraktiver sind. Dazu werden die Sauerteige nach abgeschlossener Fermentation getrocknet. Für ein hochwertiges Produkt ist es dabei unabdingbar, daß es schon in ungetrocknetem Zustand eine hohe Konzentration an Aromavorläuferstoffen und hohe Säuregrade aufweist und in reproduzierbarer Qualität hergestellt werden kann.

Die Sauerteigfermentation kann spontan oder mit Hilfe von geeigneten mikrobiologisch stabilen Starterkulturen eingeleitet werden. Spontan eingeleitete Fermentationsprozesse unterliegen jedoch einem hohen mikrobiologischen Risiko, wobei es in Folge von Fehlfermentation auch zu minderwertigen Produkten kommen kann; weiterhin sind die notwendigen Fermentationszeiten deutlich höher als bei der Verwendung einer geeigneten Starterkultur.

Starterkulturen können durch technologische "Reinkulturverfahren" unter sterilen Bedingungen in Fermentern gewonnen werden. Diese Reinkulturen sind zwar einfach und reproduzierbar zu kultivieren, aber häufig nicht mehr an die Bedingungen im Sauerteig angepaßt und daher in der Praxis unter unsterilen Bedingungen oft unsicher im Durchsetzungsvermögen, wodurch es zu qualitativen Schwankungen kommen kann. Die Herstellung von Starterkulturen durch "natürliche Reinzucht" mittels wiederholter Kultivierungs- und Rekultivierungszyklen im Lebensmittel bringt dagegen Kulturen hervor, die in der Regel besonders gut an die Milieubedingungen der Sauerteigfermentation angepaßt sind.

Aus dem Stand der Technik sind einige Herstellungsverfahren für Brot auf Sauerteigbasis bekannt; zu erwähnen ist hier die US-A-5,700,684. Darin wird die Herstellung einer Biomasse aus Laktobazillen und Hefe beschrieben, wobei eine vorherige Abtrennung der Organismen vom Kulturmedium nicht stattfindet. Die Biomasse wird zur Brotherstellung eingesetzt. Die Schrift offenbart ein Verfahren zur Herstellung von Sauerteigen mit einer nicht adaptierten Flora, da die Stämme in der Batch-Kultur gezüchtet werden.

Des Weiteren beschreibt die EP-A-0 636 692 eine getrocknete Biomasse aus Hefen und Milchsäurebakterien. Es wird hierbei ein Starterkulturextrakt bereitgestellt, welches für die Brot- und Weinherstellung oder als probiotischer Zusatz bei der Tierfutterherstellung verwandt wird, wobei Hefen und Milchsäurebakterien nicht getrennt getrocknet, sondern vor oder während des Trocknungsprozesses miteinander versetzt und unter Bewegung getrocknet werden.

FR-A-2 715 077 beschreibt ein Herstellungsverfahren für Brot auf Sauerteigbasis, wobei vor dem Animpfen mit Milchsäurebakterien Pentosen zugeführt werden. Es handelt sich um eine relativ kurzzeitige Fermentierung bei niedrigen Temperaturen.

Die EP-A-0 953 288 bezieht sich auf einen Sauerteig, welcher bei geeigneter Lagertemperatur von vorzugsweise 4 °C mehrwöchig lagerfähig ist, und nach der Lagerung sofort als Triebmittel zur Brotherstellung eingesetzt wird. Dabei spielt die Reaktivierbarkeit der Organismen durch die Drosselung des Trockensubstanzgehalts und die Hinderung der pH-Absenkung unter 4 eine große Rolle.

EP-A-0 298 605 beschreibt eine bakterielle Zusammensetzung aus Milchsäurebakterien, die bei Umgebungstemperatur stabil bleibt und als einfach einsetzbares Inokulum zur Brotherstellung eingesetzt wird, wobei der Biomasse ein vorgetrocknetes, puderiges, inertes Material (z. B. Mehl oder Milchprodukte) zugesetzt wird.

Der Stand der Technik bezieht sich auf die Bereitstellung von Biomassen, die zur Herstellung von Brotteigen herangezogen werden.

Anhand der Fermentationsbedingungen und Beschaffenheit können Sauerteige in drei Klassen eingeteilt werden (Böcker, G., P., Stolz & W. P., Hammes (1995). Neue Erkenntnisse zum Ökosystem Sauerteig und zur Physiologie der sauerteigtypischen Stämme Lactobacillus sanfrancisco und Lactobacillus pontis. Getreide, Mehl und Brot 49/6, 370-374; Stolz, P. (1999). Handbuch Sauerteig. Behr's Verlag Hamburg, 35-60. Mikrobiologie des Sauerteiges. In Spicher G. and Stephan H. (Eds.)):

Typ-l-Sauerteige sind Sauerteige oder pastöse Sauerteigstarter-Präparate aus fortlaufend geführten Teigen, deren Bakterienflora in erster Linie von heterofermentativen Laktobazillen der Spezies *Lactobacillus sanfranciscensis* und *Lb*. *pontis* dominiert werden.

Typ-II-Sauerteige sind flüssige Sauerteige, die über einen Zeitraum von mehreren Tagen fermentiert werden. Durch wiederholtes Anfrischen und einer fortlaufenden Führungsweise stellen sich in Abhängigkeit der Temperatur und der verwendeten Rohstoffe selektive Bedingungen ein, die das Wachstum von homofermentativen Stämmen der Spezies *Lb. crispatus*, *Lb. amylovorus*, *Lb. farciminis* und heterofermentativen Stämmen der Spezies *Lb. pontis* und *Lb. reuteri*, sowie *Lb. pontis-* und *Lb*. *fermentum-*ähnlichen Species begünstigen.

Bei Typ-III-Sauerteigen handelt es sich um getrocknete Sauerteige oder Sauerteigstarter-Präparate in Pulverform, deren Flora in erster Linie aus trocknungsresistenten Organismen wie z.B. *Lb. plantarum, Lb. brevis* sowie *Pediococcus pentosaceus* bestehen.

Die Säuerung bei Sauerteigen basiert auf dem Stoffwechsel von Milchsäurebakterien, im wesentlichen Laktobazillen, die aufgrund ihrer Stoffwechseleigenschaften als homo- oder heterofermentative Laktobazillen klassifiziert werden.

Homofermentative Laktobazillen verstoffwechseln Glucose fast ausschließlich zu Milchsäure. Sauerteige, die mit Hilfe bestimmter homofermentativer Laktobazillen hergestellt werden, können hohe Säuregrade erreichen. So beschreibt die DE 195 30 890 ein Verfahren zur Herstellung getrockneter Sauerteigkonzentrate unter Verwendung eines in Reinkultur gezüchteten homofermentativen *Lactobacillus* Stammes mit der Bezeichnung *Lb*. *amylovorus* SB 33, dessen Anpassung an hohe Temperaturen hohe Säuregrade liefert.

Heterofermentative Laktobazillen vergären Glucose aufgrund einer anderen Enzymaussstattung zu Milchsäure, CO₂ und Ethanol oder Essigsäure. Mit heterofermentativen Laktobazillen hergestellte Sauerteige weisen in der Regel geringere Säuregrade auf als Sauerteige, die mit homofermentativen Laktobazillen hergestellt wurden, sie sind jedoch den mit homofermentativen Laktobazillen hergestellten Sauerteigen in sensorischer Hinsicht deutlich überlegen (wozu die Produktion der Aromaträger Ethanol und Essigsäure beiträgt).

Um ein qualitativ herausragendes Sauerteigprodukt mit hohem Säuregrad und hohem Aromapotential herzustellen, wäre es wünschenswert, die vorteilhaften Eigenschaften beider Gruppen durch gezielten Einsatz gemischter Kulturen miteinander zu kombinieren. Aufgrund der unterschiedlichen Anforderungen von homo- und heterofermentativen Laktobazillen an die Kulturbedingungen, insbesondere was die Temperatur und Säureresistenz betrifft, war dies jedoch bislang noch nicht möglich:

Zum Erzielen hoher Säuregrade (Sr°) von über 160 im getrockneten Konzentrat ist die Fermentierung durch homofermentative Laktobazillen bei für die Sauerteigfermentation ungewöhnlich hohen Temperaturen nötig (siehe auch DE 195 30 890); bislang ist jedoch keine heterofermentative Spezies bekannt, die solche Temperaturbedingungen toleriert und zum Erhalt eines vollwertigen Aromas cokultiviert werden konnte.

Der Erfindung liegt die Aufgabe zugrunde, ein Verfahren zu schaffen, das durch Einsatz einer Laktobazillenflora die reproduzierbare Herstellung eines Sauerteig-Produktes mit hohen Säuregraden und gutem Aroma erlaubt. Weitere Aufgabe der Erfindung ist die Bereitstellung von dafür besonders geeigneten Laktobazillen.

Diese Aufgabe wird erfindungsgemäß durch ein Verfahren zur Herstellung eines Sauerteiges unter Verwendung von Milchsäurebakterien (Laktobazillen) gelöst, welches dadurch gekennzeichnet ist, daß der Teigmasse zur Herstellung des Sauerteiges eine adaptierte Mischflora aus hetero- und homofermentativen Laktobazillen zugesetzt wird.

Diese adaptierte Mischflora setzt sich aus Temperatur- und säureresistenten Organismen zusammen, welche durch gezielte natürliche Züchtung an die jeweiligen Fermentationsbedingungen bestens angepasst sind. Es hat sich überraschenderweise gezeigt, daß ein wesentlicher Bestandteil dieser Bakterienflora heterofermentative Laktobazillen sind, die ungewöhnlich hohe Temperaturen und niedrige pH-Werte tolerieren. Dadurch wird erstmals die Herstellung von Sauerteigen und Sauerteig-Produkten möglich, die ein hohes Aromapotential sowie extrem hohe Säuregrade aufweisen.

In einer bevorzugten Ausführungsform besteht ein solches Verfahren aus mehreren Stufen, wobei in der ersten Stufe eine erfindungsgemäße adaptierte Mischflora (Starterkultur) aus homo- und heterofermentativen Laktobazillen erzeugt wird, die in einer weiteren Verfahrensstufe (Hauptprozeß) zur Herstellung eines Sauerteiges, vorzugsweise eines Typ-II-Sauerteiges mit hohen Säuregraden eingesetzt wird. Die Mischflora des reifen Sauerteiges besteht dabei zu 50% und mehr aus heterofermentativen und zu 50% und weniger aus homofermentativen Laktobazillen.

Die erfindungsgemäße adaptierte bakterielle Mischflora enthält vorzugsweise wenigstens eine, insbesondere drei oder mehr der folgenden, zum Teil bisher nicht beschriebenen Spezies:

| | |
|---|---|
| *Lb*. *amylovorus* TMW 1.653, | unter DSM 13142 am 16.11.99 nach Budapester Vertrag bei DSMZ hinterlegt (Cluster I). |
| *Lb. species* TMW 1.652, | unter DSM 13184 am 10.12.99 nach Budapester Vertrag bei DSMZ hinterlegt (Cluster II). |
| *Lb*. *species* TMW 1.665, | unter DSM 13143 am 16.11.99 nach Budapester Vertrag bei DSMZ hinterlegt (Cluster III). |
| *Lb. pontis* TMW 1.682, | unter DSM 13144 am 16.11.99 nach Budapester Vertrag bei DSMZ hinterlegt (Cluster IV). |
| *Lb*. *reuteri* TMW 1.656, | unter DSM 13185 am 10.12.99 nach Budapester Vertrag bei DSMZ hinterlegt (Cluster V). |

In einer bevorzugten Ausführungsform wird eine adaptierte bakterielle Mischflora aus den folgenden Spezies eingesetzt:

| | |
|---|---|
| *Lb. amylovorus* TMW 1.653, | unter DSM 13142 am 16.11.99 nach Budapester Vertrag bei DSMZ hinterlegt (Cluster I); |
| *Lb. species* TMW 1.665, | unter DSM 13143 am 16.11.99 nach Budapester Vertrag bei DSMZ hinterlegt (Cluster III); |
| *Lb*. *pontis* TMW 1.682, | unter DSM 13144 am 16.11.99 nach Budapester Vertrag bei DSMZ hinterlegt (Cluster IV); |
| *Lb*. *reuteri* TMW 1.656, | unter DSM 13185 am 10.12.99 nach Budapester Vertrag bei DSMZ hinterlegt (Cluster V). |

Als besonders vorteilhaft hat sich dabei eine Zusammensetzung aus 50 bis 90% *Lb*. *amylovorus* DSM 13142, 5 bis 30% *Lb*. *species* DSM 13143, 5 bis 30% *Lb.pontis* DSM 13144 und 0 bis 15% *Lb*. *reuteri* DSM 13185 erwiesen.

Insbesondere werden diese Spezies oder Mischfloren als Starterkulturen für die Sauerteigherstellung oder zur Herstellung von Sauerteig-Starterkulturen eingesetzt.

Hinsichtlich eines wachsenden Bedarfs an aromareichen natürlichen Teigsäuerungsmitteln in der Backmittelindustrie und industriellen Bäckereien, sowie wachsender Verbraucheransprüche in Bezug auf die geschmackliche Qualität von Nahrungsmitteln und ökologischer Verfahrensweisen bei der Nahrungsmittelherstellung, ist es notwendig, auf natürlichem Wege hochwertige Sauerteig-Produkte in reproduzierbarer Weise herstellen zu können. Die Verwendung der erfindungsgemäßen adaptierten bakteriellen Mischflora gewährleistet auf natürlichem Wege alle für einen qualitativ hochwertigen Sauerteig wünschenswerten Fermentations-Vorraussetzungen:

Der Einsatz der erfindungsgemäßen bakteriellen Mischfloren unter erhöhten Temperaturbedingungen vermindert die Gefahr von Fehlfermentationen, auch bei Verwendung unterschiedlicher Rohstoffe. Die Vielzahl der in der Mischflora vorkommenden, und an hohe Temperaturen sowie niedrige pH-Werte angepassten Stämme, stellt aufgrund unterschiedlicher Substratvorlieben und Toleranzeigenschaften sicher, daß sich die Mehrzahl der eingesetzten Stämme, zumindest aber einer, durchsetzt.

Durch ein schnelles Absinken des pH-Wertes in der ersten Phase des Fermenationsprozesses aufgrund der schnellen Säureproduktion der homofermentativen Spezies, ist ein schnelles Verdrängen der bei Getreidefermentationen immer vorhandenen Spontanflora und somit eine Vermeidung von Fehlfermentationen gewährleistet. Weiterhin ermöglicht die hohe Säure- und Temperaturresistenz der heterofermentativen Spezies das Erreichen einer sehr hohen Endkonzentration an Säuren und Aromavorläuferstoffen, da die heterofermentativen Stämme trotz der durch den homofermentativen Stoffwechsel bereits erreichten hohen Säuregrade, auch nach abgeschlossener homofermentativer Gärung noch weiter stoffwechselaktiv sind. So wird auf natürliche Weise die reproduzierbare Produktion eines qualitativ hochwertigen Sauerteig-Produktes erreicht, das hohe Säuregrade mit vollwertigem Aroma kombiniert.

Durch den Einsatz einer mikrobiologisch definierten Bakterienflora, die an die Bedingungen insbesondere dieser Sauerteigfermentation bestens adaptiert ist, kann eine gleichbleibend hohe Qualität der Sauerteig-Produkte gewährleistet werden, da diese adaptierten Mischkulturen eine hohe Durchsetzungsfähigkeit aufweisen. Darüber hinaus kann die genaue mikrobielle Zusammensetzung jederzeit durch modernste molekularbiologische Analysenverfahren (z. B. PCR-Methoden) bestimmt und überprüft werden. Auf diese Weise wird insbesondere den mikrobiologischen Anforderungen an eine sichere Lebensmittelherstellung Rechnung getragen.

Zur Herstellung einer breiten Palette an Sauerteigen, die sich hinsichtlich Aroma und/oder Säuregehalt unterscheiden, können die oben genannten Spezies auch alleine oder in anderer als bevorzugter Kombination zur Sauerteigherstellung eingesetzt werden. Weiterhin können zusätzlich zur bakteriellen Mischflora auch Hefen zur Fermentation eingesetzt werden.

Die Herstellung verschiedener Varianten von Sauerteigen mit hohen Säuregraden und ausgewogenem Aroma erfolgt vorzugsweise durch Einsatz eines mehrstufigen Verfahrens: In einer ersten Stufe erfolgt die Herstellung der erfindungsgemäßen adaptierten Mischflora, welche in einer zweiten Verfahrensstufe (dem Hauptprozeß) zur Herstellung eines an Aromavorläufern reichen hochsäurehaltigen Sauerteiges eingesetzt wird. Die Mikroflora dieses Sauerteiges setzt sich dabei vorzugsweise zu 50% und mehr aus heterofermentativen und zu 50% und weniger aus homofermentativen Laktobazillen zusammen. Zur Herstellung getrockneter Sauerteig-Produkte schließt sich eine weitere Verfahrensstufe an, bei der die Trocknung durch gängige Trocknungsverfahren, wie Walzen-, Sprüh- oder Vakuumtrocknung erfolgt.

Die Herstellung einer mikrobiologisch stabilen adaptierten Mischflora (Starterkultur) erfolgt erfindungsgemäß durch kontinuierliche Propagation eines mit den oben genannten Stämmen inokulierten Getreidemahlprodukt-WasserGemisches bei Temperaturen zwischen 32 und 46°C, vorzugsweise bei 35 bis 44°C über wiederholte Zeitintervalle von 30 bis 60, vorzugsweise zwischen 40 bis 55, insbesondere 48 Stunden.

Der Hauptprozeß ist dabei vorzugsweise ein Verfahren zur Herstellung eines Typ-II-Sauerteiges, bei dem ein Getreidemahlprodukt-Wasser-Gemisch mit der erfindungsgemäßen Starterkultur inokuliert und bei Temperaturen zwischen 25 bis 46°C, vorzugsweise zwischen 32 und 46°C fermentiert wird.

Gemäß einer besonders bevorzugten Ausführungsweise wird das Hauptverfahren bei Fermentationstemperaturen zwischen 35 bis 46°C, vorzugsweise zwischen 39 und 44°C begonnen. Zum Ende hin werden Temperaturen zwischen 25 bis 39°C, vorzugsweise 32 bis 37°C, eingehalten.

Die Inkubationszeit des Hauptverfahrens wird dabei erfindungsgemäß so gewählt, daß am Ende der Anteil heterofermentativer Laktobazillen an der bakteriellen Flora 50% oder mehr, vorzugsweise über 75%, besonders bevorzugt 90% und insbesondere zwischen 95 bis 100% beträgt.

Als zweckmäßig hat sich insbesondere der Einsatz eines Hauptverfahrens herausgestellt, welches 2 bis 8 Tage, vorzugsweise 3-7 Tage, in besonders bevorzugter Weise 4-5 Tage andauert.

Für den Erhalt einer solchen Flora am Ende des Hauptverfahrens ist die Wahl einer geeigneten Inkubationstemperatur während des Hauptverfahrens von besonderer Bedeutung, insbesondere auch um das Wachstum unerwünschter Mikroorganismen zu unterdrücken. So kann mit Hilfe einer geeigneten Inkubationstemperatur im Hauptverfahren eine Bakterienflora selektiert werden, die am Ende des Hauptverfahrens zu 80 bis 100%, insbesondere 95 bis 100% aus heterofermentativen Laktobazillen, vorzugsweise Lb. species (DSM 13143) besteht.

Ein besonders geeignetes Beispiel eines so hergestellten Sauerteiges ist ein Typ-II-Sauerteig, der zum Ende hin zu 80 bis 100%, vorzugsweise 95 bis 100% heterofermentative Laktobazillen, insbesondere *Lb. species* (DSM 13143) enthält.

In einer vorteilhaften Ausführungsform wird das Verfahren zur Herstellung von Typ-II-Sauerteigen durch Inokulierung der Ausgangsprodukte mit einer erfindungsgemäßen Starterkultur eingeleitet. Das anschließende Hauptverfahren wird bei Temperatur zwischen 39 und 44°C eingeleitet, die zum Ende hin auf zwischen 32 bis 35°C abfallen und über zwei bis acht Tage durchgeführt. Das Endprodukt dieser vorteilhaften Ausführungsform besteht zu 95 bis 100% aus Lb.species (DSM 13143).

Gemäß einer besonders bevorzugten Ausführungsform der Erfindung, wird die erfindungsgemäße Mischflora zunächst in oben genannter Weise erzeugt, in den oben genannten Hauptprozeß zu einem Typ-II-Sauerteig vergoren und anschließend durch gängige Verfahren getrocknet. Zur Herstellung von getrockneten Sauerteigen mit besonders röstig-malzigem Aroma hat sich hierbei die Verwendung eines Walzentrocknungsverfahrens als besonders geeignet erwiesen.

Als Getreidemahlprodukte können dabei prinzipiell alle Sorten von Getreidemahlprodukten, insbesondere Roggen- oder Weizen-Mahlprodukte oder Mischungen daraus mit Aschegehalten zwischen 1000 bis 5000 mg/100g eingesetzt werden.

Weiterhin können dem Fermentationsansatz oder dem fertigen Sauerteig ein oder mehrere Enzyme (z. B. Amyloglucosidasen, Pentosanasen) oder Mehle beigemengt werden, um die Fermentation, das Aroma und/oder die Trocknung günstig zu beeinflussen. Vorzugsweise werden Amyloglucosidasen eingesetzt, um Unterschiede in der Rohwaren-Qualität auszugleichen und die Fermentation zu unterstützen.

Die Erfindung soll nachfolgend anhand von Bespielen erläutert werden; dabei wird zunächst eine allgemeine Beschreibung gegeben:

Die Herstellung verschiedener Varianten von getrockneten Sauerteigen mit hohen Säuregraden erfolgt durch Kombination eines Verfahrens zur Herstellung einer stabilen adaptierten Mischkultur mit der Herstellungsweise für Typ-II-Sauerteige, unter Verwendung von Roggen- und Weizenmahl-Produkten mit einem Aschegehalt von 1000-5000 mg/100g, Wasser (Teigausbeute 250 - 600) und Anstellgut (stabile Mischflora) bei Temperaturen zw. 25 - 43°C und einer Fermentationszeit von 2-8 Tagen und einem anschließenden Trocknungsverfahren.

### Beispiel 1: Herstellung der stabilen adaptierten Mischkultur zur Herstellung von hochsäurehaltigen Sauerteigen oder Sauerteig-Produkten:

Durch kontinuierliche Propagation eines Getreidemahlprodukt-Wasser Gemisches bei 35 bis 46°C, vorzugsweise zwischen 39 und 44°C in einem Rhythmus von 30 bis 60, vorzugsweise zwischen 40 bis 55, insbesondere 48 Stunden, unter sonst konstanten Bedingungen (Rohstoffe, Teigausbeute, Impfgutmenge) wird die für die jeweiligen Bedingungen bestgeeignete Mikroflora (Milchsäurebakterien / Milchsäurebakterien und Hefen) selektiert und als Impfgut zur Herstellung von Sauerteigen bzw. Sauerteig-Produkten verwendet.

### Beispiel 2a: Herstellung eines getrockneten Roggen- / Weizen-Sauerteiges mit 150-180 Sr°:

Mit einer kontinuierlich propagierten Mischkultur bestehend aus *Lb*. *pontis*, *Lb*. *species*, *Lb*. *reuteri* und *Lb*. *amylovorus* Stämmen wird ein Teig angeimpft, bei Temperaturen zwischen 38 bis 43°C 2 bis 8 Tage fermentiert und anschließend mit üblichen Verfahren getrocknet. Die Trocknung kann durch Walzentrocknung, Sprühtrocknung oder Vakuumtrocknung erfolgen. Der auf diese Weise hergestellte getrocknete Sauerteig weist einen Trockensubstanzgehalt von 92-98 % auf und enthält einen hohen Gehalt an aromawirksamen Inhaltsstoffen und fermentativ gebildeten organischen Säuren, die eine niedrige Dosierung von 2-8% (bezogen auf Mehl) in der Backware ermöglichen. Die so hergestellten getrockneten Sauerteige können zur direkten Herstellung von Roggen, Roggenmisch-, Weizenmisch-, Weizen-Gebäcken, bzw. SauerteigGebäcken oder als Ingredienz für Fertigmehl- und Backmischungen verwendet werden.

### Beispiel 2b: Herstellung eines getrockneten Roggen-/ Weizen-Sauerteig-Produktes mit 180-230 Sr°:

Mit einer kontinuierlich propagierten Mischkultur aus *Lb*. *pontis, Lb*. *species*, *Lb*. *reuteri* und *Lb*. *amylovorus* wird ein Teig angeimpft. Zur Unterstützung des Stärkeabbaus wird dem Ansatz eine Glucoamylase (0,5-1,5 ‰, bezogen auf Roggen-/Weizenmahl-Produkte) zugegeben. Der Ansatz wird ebenfalls bei Temperaturen zw. 38-43°C 4-8 Tage fermentiert und anschließend mit üblichen Verfahren getrocknet. Die Trocknung kann durch Walzentrocknung oder Sprühtrocknung erfolgen. Das auf diese Weise hergestellte Sauerteig-Produkt weist einen Trockensubstanzgehalt von 92-98 % auf und enthält einen hohen Gehalt an aromawirksamen Inhaltsstoffen und fermentativ gebildeten organischen Säuren, die eine niedrige Dosierung von 1-8% (bezogen auf Mehl) in der Backware ermöglichen. Die so hergestellten getrockneten Sauerteige oder Sauerteigprodukte können zur direkten Herstellung von Roggen, Roggenmisch-, Weizenmisch- und Weizengebäcken, sowie als Ingredienz für Fertigmehl- und Backmischungen verwendet werden.

Beispielhaft wird nachfolgend die Analyse eines industriellen Fermentationsprozesses eines Roggensauerteiges erläutert:

### Beispiel 3: Analyse eines industriellen Fermentationsprozesses

Im Verlauf eines Fermentationsprozesses über einen Zeitraum von 5 Tagen, der mit einer Mischkultur aus den oben genannten Stämmen beimpft war, wurden täglich Proben entnommen und mikrobiologisch sowie chemisch (pH-Wert, Säuregrad, HPLC-Analyse) analysiert. Die direkt nach der Beimpfung entnommenen Proben ergaben auf einem modifizierten MRS-Agar eine Milchsäurebakterien-Keimzahl von 1,38 x 10⁸ KbE/g (Kolonie bildende Einheiten pro Gramm Sauerteig). Der pH-Wert und der Säuregrad lagen zu diesem Zeitpunkt bei 5,8, die Temperatur des Sauerteiges betrug 42,2°C. Nach 24 Stunden war eine Milchsäurebakterien-Keimzahl von 2,6 x 10⁹ KbE/g erreicht. 5 Stunden später wurde der Minimum-pH-Wert von 3,4 gemessen. Nach 5 Tagen wurde der maximale Säuregrad von 46 Sr° bestimmt, wobei die Milchsäurebakterien-Keimzahl auf 3,2 x 10⁸ KbE/g gefallen war. Die Temperatur nach 2 Tagen lag noch oberhalb von 40°C und fiel bis zum Ende der Fermentation auf 34,4°C ab.

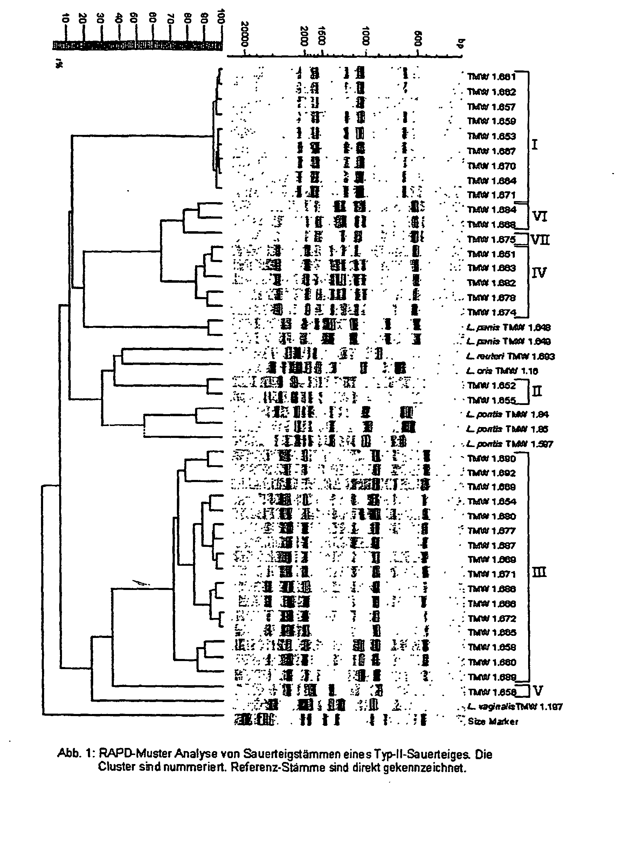

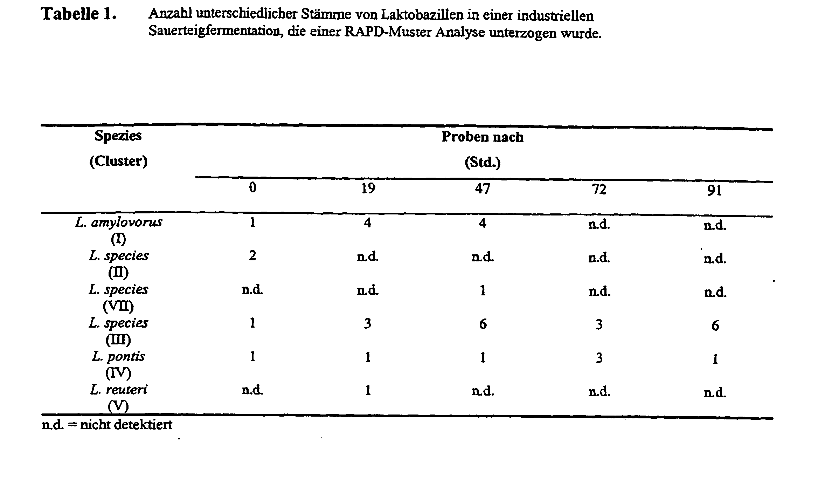

Um einen ersten Überblick über die Fermentationsflora zu bekommen, wurden im Verlaufe des Fermentationsprozesses 36 Milchsäurebakterien-Stämme isoliert und einer RAPD-Analyse unterzogen (Abb.1). Die RAPD-Muster wurde mit denen von Referenzstämmen, die man in solchen Fermentationsprozessen erwarten könnte, verglichen. Die RAPD-Muster zeigten 3 bis 6 Hauptfragmente mit einer Größe von 3,5 bis 0,5 kb. Unter Anwendung eines speziellen Algorithmus konnten die isolierten Stämme in 7 verschiedene Cluster eingeordnet werden. Die Homogenität innerhalb der Cluster war unterschiedlich hoch. Zum Beispiel beinhaltet Cluster I Stämme der *Spezies Lb*. *amylovorus*, die Homogenität innerhalb des Clusters I lag bei 96%. Die Homogenität innerhalb der anderen Cluster lag bei 70-90%. Für eine zuverlässige Identifizierung, wurden von mindestens einem Repräsentant eines Clusters 520 bp vom 5'-Ende her sequenziert. Die Stämme von Cluster I konnten hiermit als *Lb. amylovorus* identifiziert werden, einer obligat homofermentativen Milchsäurebakterien-Spezies. Die Stämme der Cluster II, III und VII konnten keiner bisher bekannten Bakterienspezies zugeordnet werden und nahmen eine phylogenetische Position zwischen *Lb*. *pontis* und *Lb*. *vaginalis* ein. Die gefundenen Spezies dieser Cluster waren alle heterofermentativ. Zur genaueren Untersuchung dieser bisher unbekannten Spezies wurde die komplette 16S rDNA von *Lb. spezies* TMW 1,655 und von *Lb*. *spezies* TMW 1,666 sequenziert. Cluster IV und VI sind heterofermentative *Lb. pontis* Stämme. Cluster V bestand aus einem einzelnen Stamm der heterofermentativen Spezies *Lb*. *reuteri.* Eine RAPD-Analyse der Gesamtflora einer Sauerteigprobe (46 Kolonien einer Agarplatte einer 10⁻⁸ Verdünnung) nach 48 Stunden Fermentationszeit ergab 63% *Lb*. *amylovorus*, 15% *Lb*.*pontis* und 15% *Lb*. *species* und 7% *Lb*. *reuteri*. Tab. 1 zeigt die Florenzusammensetzung im Verlauf der gesamten Fermentationszeit.

Die Untersuchung von Sauerteigproben aus demselben Sauerteigprozess 2 Jahre nach der ersten Isolierung, ergab ein vergleichbares Resultat in der Stamm-Zusammensetzung, sogar die RAPD-Muster waren vergleichbar.

Die nachfolgend aufgeführte Abbildung und Tabelle dienen zur Veranschaulichung der obigen Beispiele. Es stellen dar:

| | |
|---|---|
| Abbildung 1 | die RAPD-Musteranalyse der 36 isolierten Sauerteigstämme im Vergleich mit Referenzstämmen; |
| Tabelle 1 | die Anzahl unterschiedlicher SauerteigStämme im Verlauf eines erfindungsgemäßen Fermentationsprozesses mit Hilfe einer RAPD-Muster Analyse. |

Im Gegensatz zur DE 195 30 890 A1, in der eine Reinkultur von *Lb. amylovorus* zur Herstellung von hochsäurehaltigen getrockneten Sauerteigkonzentraten beschrieben wird, wird im erfindungsgemäßen Verfahren ein natürliches Zuchtverfahren benutzt, um geeignete und stabile Mikrofloren herzustellen, die die Produktion von stark säurehaltigen Sauerteigen und Sauerteig-Produkten ermöglichen, die außerdem hohe aromatische Qualität aufweisen.

## Patentansprüche

1. Verfahren zur Herstellung eines Sauerteiges unter Verwendung von Milchsäurebakterien, **dadurch gekennzeichnet, dass** der Teigmasse zur Herstellung des Sauerteiges eine adaptierte Mischflora aus hetero- und homofermentativen Laktobazillen, welche durch "natürliche Reinzucht" mittels wiederholter Kultivierungs- und Rekultivierungszyklen im Lebensmittel gewonnen werden, zugesetzt wird, wobei das Verfahren aus mehreren Stufen besteht, in denen in einer ersten Stufe eine adaptierte Mischflora (Starterkultur) aus heterofermentativen und homofermentativen Laktobazillen erzeugt wird, die in einer weiteren Verfahrensstufe (Hauptprozess) zur Herstellung eines Sauerteiges, vorzugsweise eines Typ-II-Sauerteiges mit hohen Säuregraden, eingesetzt wird, dessen Mikroflora zu 50% und mehr aus heterofermentativen und zu 50% und weniger aus homofermentativen Laktobazillen besteht.

2. Verfahren nach Anspruch 1, **dadurch gekennzeichnet, dass** Getreidemahlprodukte mit einem Aschegehalt von 1000 bis 5000 mg/100 g verwendet werden.

3. Verfahren nach einem der vorhergehenden Ansprüche, **dadurch gekennzeichnet, dass** als Getreidemahlprodukte Roggen- oder Weizenmahlprodukte oder Mischungen daraus eingesetzt werden.

4. Verfahren nach einem der vorhergehenden Ansprüche, **dadurch gekennzeichnet, dass** die Herstellung der Starterkultur durch kontinuierliche Propagation eines mit einer Mischflora aus homo- und heterofermentativen Laktobazillen inokulierten Getreideprodukt-Wassergemisches über wiederholte Zeitintervalle von zwischen 30 bis 60, vorzugsweise 40 bis 55 und besonders bevorzugt 48 Stunden bei Temperaturen zwischen 32 und 46°C und vorzugsweise zwischen 35 bis 44°C erfolgt, bis eine mikrobiologisch stabile Mischflora (Starterkultur) entsteht.

5. Verfahren nach einem der vorhergehenden Ansprüche, **dadurch gekennzeichnet, dass** die adaptierte bakterielle Mischflora wenigstens einen der Stämme *Lb*. *amylovorus* DSM 13142, *Lb*. *species* DSM 13143, *Lb*. *species* 13184, *Lb*. *pontis* DSM 13144 und *Lb*. *reuteri* DSM 13185, vorzugsweise 50 bis 90% *Lb. amylovorus* DSM 13142, 5 bis 30% *Lb. species* DSM 13143, 5 bis 30% *Lb.pontis* DSM 13144 und 0 bis 15% *Lb*. *reuteri* DSM 13185 enthält.

6. Bakterielle Mischflora der Stämme *Lb. amylovorus* DSM 13142, *Lb. species* DSM 13143, *Lb. species* 13184, *Lb. pontis* DSM 13144 und *Lb. reuteri* DSM 13185.

7. Verfahren nach einem der Ansprüche 1 bis 5, **dadurch gekennzeichnet, dass** die Inkubationstemperatur des Hauptprozesses zwischen 25 bis 46°C und vorzugsweise zwischen 32 und 46°C liegt.

8. Verfahren nach Anspruch 7, **dadurch gekennzeichnet, dass** der Hauptprozess mit Fermentationstemperaturen zwischen 35 bis 46°C und vorzugsweise zwischen 39 bis 44°C beginnt und bis zum Ende auf Temperaturen zwischen 25 bis 39°C, vorzugsweise zwischen 32 bis 37°C abfällt.

9. Verfahren nach einem der Ansprüche 1 bis 5 und 7 bis 8, **dadurch gekennzeichnet, dass** die Inkubationszeit des Hauptverfahrens so gewählt wird, dass am Ende des Hauptverfahrens der Anteil der heterofermentativen Laktobazillen an der Mischflora 50% oder mehr, vorzugsweise über 75%, besonders bevorzugt über 90% und insbesondere zwischen 95 und 100% beträgt.

10. Verfahren nach einem der Ansprüche 1 bis 5 und 7 bis 9, **dadurch gekennzeichnet, dass** die Inkubationszeit des Hauptverfahrens 2 bis 8 Tage beträgt.

11. Verfahren nach einem der Ansprüche 1 bis 5 und 7 bis 10, **dadurch gekennzeichnet, dass** die Inkubationstemperatur des Hauptverfahrens so gewählt wird, dass die bakterielle Mischflora am Ende des Hauptverfahrens zu 80 bis 100% und vorzugsweise zu 95 bis 100% aus heterofermentativen Laktobazillen, vorzugsweise *Lb. species* DSM 13143 besteht.

12. Verfahren zur Herstellung von Typ-II-Sauerteigen nach einem der Ansprüche 1 bis 5 und 7 bis 11, **dadurch gekennzeichnet, dass** es durch Inokulierung einer Starterkultur nach Anspruch 6 eingeleitet und über 2 bis 8 Tage durchgeführt wird, mit einer Anfangstemperatur des Hauptverfahrens zwischen 39 bis 44°C, die zum Ende auf zwischen 32 bis 37°C abfällt, welches in einem Endprodukt resultiert, das zu 95 bis 100% aus *Lb. species* DSM 13143 besteht.

13. Sauerteig, insbesondere Typ-II-Sauerteig mit einer bakteriellen Flora, die zu 80 bis 100% und vorzugsweise zu 95 bis 100% *Lb. species* DSM 13143 enthält.

14. Verfahren zur Herstellung von getrockneten Sauerteigen/Sauerteig-Produkten, **dadurch gekennzeichnet, dass** sich an ein Verfahren nach einem der Ansprüche 1 bis 5 und 7 bis 12 ein Trocknungsvorgang anschließt.

15. Verfahren nach Anspruch 14, **dadurch gekennzeichnet, dass** die Trocknung durch Walzen-, Sprüh- oder Vakuumtrocknung, vorzugsweise Walzentrocknung erfolgt.

16. Verwendung einer bakteriellen Mischflora nach Anspruch 6 als Starterkultur für die Herstellung von Sauerteigen oder von Sauerteigstarterkulturen.

17. Stämme der Spezies *Lb. species* DSM 13143, *Lb. pontis* DSM 13144, *Lb. amylovorus* DSM 13142, *Lb*. *reuteri* DSM 13185 sowie *Lb*. *species* DSM 13184 jeweils allein oder in beliebiger Kombination miteinander oder mit anderen Mikroorganismen.

18. Verfahren nach einem der Ansprüche 1 bis 5, 7 bis 12 oder 14, **dadurch gekennzeichnet, dass** im Hauptfermentationsprozess ein oder mehrere Enzyme, vorzugsweise Amyloglucosidasen beigemengt werden, um die Fermentation zu unterstützen.

## Claims

1. A method of producing a sour dough using lactic acid bacteria, **characterised in that** an adapted mixed flora of heterofermentative and homofermentative lactobacilli, which have been produced by "natural pure breeding" by means of repeated cultivation and recultivation cycles in foodstuffs, is added to the dough composition to produce the sour dough, the method comprising a plurality of stages, in which, in a first stage, an adapted mixed flora (starter culture) of heterofermentative and homofermentative lactobacilli is produced, which is used in a further method stage (main process) for producing a sour dough, preferably a type II sour dough with high degrees of acidity, the microflora of which comprises heterofermentative lactobacilli in an amount of 50% and more and homofermentative lactobacilli in an amount of 50% and less.

2. A method as claimed in Claim 1, **characterised in that** milled grain products are used with an ash content of 1,000 to 5,000 mg/100g is used.

3. A method as claimed in one of the preceding claims, **characterised in that** milled rye or wheat products or mixtures thereof are used as the milled grain products.

4. A method as claimed in one of the preceding claims, **characterised in that** the production of the starter culture is effected by continuous propagation of a grain product-water mixture inoculated with a mixed flora of homofermentative and heterofermentative lactobacilli over repeated time intervals of between 30 to 60, preferably 40 to 55 and particularly preferably 48 hours at temperatures between 32 and 46°C and preferably between 35 and 44°C, until a microbiologically stable mixed flora (starter culture) is produced.

5. A method as claimed in one of the preceding claims, **characterised in that** the adapted bacterial mixed flora includes at least one of the strains Lb. amylovorus DSM 13142, Lb. species DSM 13143, Lb. species 13184, Lb. pontis DSM 13144 and Lb. reuteri DSM 13185, preferably 50 to 90% Lb. Amylovorus DSM 13142, 5 to 30% Lb. species DSM 13143, 5 to 30% Lb. pontis DSM 13144 and 0 to 15% Lb. reuteri DSM 13185.

6. A bacterial mixed flora of the strains Lb. amylovorus DSM 13142, Lb. species DSM 13143, Lb. species 13184, Lb. pontis DSM 13144 and Lb. reuteri DSM 13185.

7. A method as claimed in one of Claims 1 to 5, **characterised in that** the incubation temperature of the main process is between 25 to 46°C and preferably between 32 and 46°C.

8. A method as claimed in Claim 7, **characterised in that** the main process begins with fermentation temperatures between 25 and 46°C and preferably between 39 to 44°C and decreases by the end to temperatures between 25 to 39°C, preferably between 32 to 37°C.

9. A method as claimed in one of Claims 1 to 5 and 7 to 8, **characterised in that** the incubation time of the main method is so selected that the proportion of the heterofermentative lactobacilli in the mixed flora is 50% or more, preferably above 75%, particularly preferably above 90% and in particular between 95 and 100%, at the end of the main method.

10. A method as claimed in one of Claims 1 to 5 and 7 to 9, **characterised in that** the incubation time of the main method is 2 to 8 days.

11. A method as claimed in one of Claims 1 to 5 and 7 to 10, **characterised in that** the incubation temperature of the main method is so selected that the bacterial mixed flora at the end of the main method consists of heterofermentative lactobacilli, preferably Lb. species DSM 13143, to the extent of 80 to 100% and preferably 95 to 100%.

12. A method of producing type II sour doughs as claimed in Claims 1 to 5 and 7 to 11, **characterised in that** it is initiated by inoculation of a starter culture as claimed in Claim 6 and is performed for 2 to 8 days with a starting temperature of the main method between 39 to 44°C, which decreases towards the end to between 32 to 37°C, which results in an end product which consists of Lb. species DSM 13143 to the extent of 95 to 100%.

13. A sour dough, particularly a type II sour dough, with a bacterial flora which contains up to 80 to 100% and preferably up to 95 to 100% Lb. species DSM 13143.

14. A method of producing dried sour doughs/sour dough products, **characterised in that** a drying process follows a method as claimed in one of claims 1 to 5 and 7 to 12.

15. A method as claimed in Claim 14, **characterised in that** the drying is effected by roller, spray or vacuum drying, preferably roller drying.

16. The use of a bacterial mixed flora as claimed in Claim 6 as the starter culture for the production of sour doughs or of sour dough starter cultures.

17. Strains of the species Lb. species DSM 13143, Lb. pontis DSM 13144, Lb. amylovorus DSM 13142, Lb. reuteri DSM 13185 and Lb. species DSM 13184, alone or in any desired combination with one another or with other microorganisms.

18. A method as claimed in one of Claims 1 to 5, 7 to 12 or 14, **characterised in that** one or more enzymes, preferably amyloglucosidases, are added in the main fermentation process in order to assist the fermentation.

## Revendications

1. Procédé pour la préparation d'un levain avec utilisation de bactéries lactiques, **caractérisé en ce qu'**on ajoute à la masse de pâte pour la préparation du levain une flore mixte adaptée à base de lactobacilles hétéro- et homofermentatifs, qui ont été obtenus dans l'aliment par "culture pure naturelle" au moyen de cycles de culture et de reculture répétés, tandis que le procédé comprend plusieurs étapes, dans lesquelles dans une première étape on produit à partir de lactobacilles hétérofermentatifs et homofermentatifs une flore mixte adaptée (culture de démarrage), qui est utilisée dans une autre étape opératoire (processus principal) pour la préparation d'un levain, de préférence d'un levain de type II ayant des degrés d'acidité élevés, dont la microflore consiste pour 50% et plus en des lactobacilles hétérofermentatifs et pour 50% et moins en des lactobacilles homofermentatifs.

2. Procédé selon la revendication 1, **caractérisé en ce qu'**on utilise des produits de broyage de céréales ayant une teneur en cendres de 1000 à 5000 mg/100 g.

3. Procédé selon l'une des revendications précédentes, **caractérisé en ce qu'**on utilise comme produits de broyage de céréales des produits de broyage de seigle ou de froment, ou des mélanges de ceux-ci.

4. Procédé selon l'une des revendications précédentes, **caractérisé en ce que** la préparation de la culture de démarrage s'effectue par propagation en continu d'un mélange de produit de céréales-eau inoculé avec une flore mixte de lactobacilles homo- et hétérofermentatifs, sur des intervalles de temps répétés de 30 à 60, de préférence de 40 à 55 et plus particulièrement de 48 heures, à des températures entre 32 et 46°C et de préférence entre 35 et 44°C, jusqu'à formation d'une flore mixte stable du point de vue microbiologique (culture de démarrage).

5. Procédé selon l'une des revendications précédentes, **caractérisé en ce que** la flore bactérienne mixte adaptée contient au moins l'une des souches *Lb*. *amylovorus* DSM 13142, *Lb. species* DSM 13143, *Lb*. *species* DSM 13184, *Lb. pontis* DSM 13144 et *Lb*. *reuteri* DSM 13185, de préférence 50 à 90% de *Lb. amylovorus* DSM 13142, 5 à 30% de *Lb. species* DSM 13143, 5 à 30% de *Lb. pontis* DSM 13144 et 0 à 15% de *Lb. reuteri* DSM 13185.

6. Flore bactérienne mixte de souche *Lb. amylovorus* DSM 13142, *Lb. species* DSM 13143, *Lb. species* DSM 13184, *Lb*. *pontis* DSM 13144 et *Lb. reuteri* DSM 13185.

7. Procédé selon l'une des revendications 1 à 5, **caractérisé en ce que** la température d'incubation du processus principal se situe entre 25 et 46°C et de préférence entre 32 et 46°C.

8. Procédé selon la revendication 7, **caractérisé en ce que** le processus principal débute avec des températures de fermentation entre 35 et 46°C et de préférence entre 39 et 44°C, et se déroule jusqu'à la fin à des températures entre 25 et 39°C, de préférence entre 32 et 37°C.

9. Procédé selon l'une des revendications 1 à 5 et 7 à 8, **caractérisé en ce que** la durée d'incubation du processus principal est choisie de telle manière que, à la fin du processus principal, la proportion des lactobacilles hétérofermentatifs par rapport à la flore mixte soit de 50% ou plus, de préférence supérieure à 75%, particulièrement préférablement supérieure à 90% et tout particulièrement entre 95 et 100%.

10. Procédé selon l'une des revendications 1 à 5 et 7 à 9, **caractérisé en ce que** la durée d'incubation du processus principal est de 2 à 8 jours.

11. Procédé selon l'une des revendications 1 à 5 et 7 à 10, **caractérisé en ce que** la température d'incubation du processus principal est choisie de telle manière que la flore bactérienne mixte à la fin du processus principal consiste pour 80 à 100% et de préférence pour 95 à 100% en des lactobacilles hétérofermentatifs, de préférence en *Lb. species* DSM 13143.

12. Procédé pour la préparation de levains de type II selon l'une des revendications 1 à 5 et 7 à 11, **caractérisé en ce qu'**il est amorcé par inoculation d'une culture de démarrage selon la revendication 6 et est mis en oeuvre sur 2 à 8 jours, avec une température de départ du processus principal entre 39 et 44°C, qui descend à la fin entre 32 et 37°C, ce qui donne un produit final qui consiste en 95 à 100% de *Lb. species* DSM 13143.

13. Levain, en particulier levain de type II, ayant une flore bactérienne qui contient 80 à 100%, et de préférence 95 à 100% de *Lb. species* DSM 13143.

14. Procédé pour la préparation de levains/produits de levain séchés, **caractérisé en ce qu'**une opération de séchage est reliée à un procédé selon l'une des revendications 1 à 5 et 7 à 12.

15. Procédé selon la revendication 14, **caractérisé en ce que** le séchage est effectué par séchage au tambour, par pulvérisation ou sous vide, de préférence par séchage au tambour.

16. Utilisation d'une flore bactérienne mixte selon la revendication 6 comme culture de démarrage pour la préparation de levains ou de cultures de démarrage pour levain.

17. Souches d'espèces *Lb. species* DSM 13143, *Lb. pontis* DSM 13144, *Lb. amylovorus* DSM 13142, *Lb. reuteri* DSM 13185 ainsi que *Lb. species* DSM 13184, à chaque fois seules ou dans une combinaison quelconque entre elles ou avec d'autres micro-organismes.

18. Procédé selon l'une des revendications 1 à 5, 7 à 12 ou 14, **caractérisé en ce que** dans le processus principal de fermentation on ajoute une ou plusieurs enzymes, de préférence des amyloglucosidases, pour assister la fermentation.
